(19)

**Europäisches Patentamt**

**European Patent Office**

**Office européen des brevets**

(11)  **EP 4 620 982 A1**

(12)  **EUROPEAN PATENT APPLICATION**

(43) Date of publication:
24.09.2025  Bulletin 2025/39

(21) Application number: 24305421.0

(22) Date of filing: 20.03.2024

(51) International Patent Classification (IPC):
$C08B\ 30/14^{(2006.01)}$  $C08B\ 31/12^{(2006.01)}$
$C08L\ 3/08^{(2006.01)}$  $A61K\ 8/73^{(2006.01)}$

(52) Cooperative Patent Classification (CPC):
C08L 3/08; A61K 8/732; A61Q 19/00; C08B 30/14;
C08B 31/12

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: ROQUETTE FRERES
62136 Lestrem (FR)

(72) Inventors:
• MENTINK, Léon
  59000 Lille (FR)
• LOUVET-POMMIER, Géraldine
  60190 Lachelle (FR)
• LACORE, Camille
  95160 Montmorency (FR)
• LESUR, Thomas
  59184 Sainghin en Weppes (FR)

(74) Representative: Plasseraud IP
104 Rue de Richelieu
CS92104
75080 Paris Cedex 02 (FR)

(54)  **PREGELATINIZED HYDROPHILICALLY MODIFIED LEGUME STARCH WITH INCREASED HYDROPHOBICITY AND ITS USES AS A WATER-RESISTANT FILM FORMER**

(57)  The present application provides a pregelatinized hydroxypropylated pea starch that has improved water-resistant film forming property compared to previously known film forming pea starches. The starch may be characterized by a RVA viscosity profile. It may be obtained by the pregelatinization of a hydroxypropylated pea starch, notably on a drum dryer. The improved water-resistance was illustrated on films immersed into water and was also characterized by the determination of the hydrophobicity value of the starch using force tensiometry, goniometry and the Young-Laplace and Fowkes model.

EP 4 620 982 A1

## Description

### Technical Field

The invention pertains to the field of film forming modified starches, which form water-resistant films.

### Background Art

Film forming ingredients that are both able to withstand dissolution into water a time long enough to suit the targeted use, but also that are either removable by water or dissolved ultimately by acidic or basic aqueous medium, are looked for in many applications in different fields.

In the field of cosmetics, waterproof cosmetics need, on the one hand, that the film formed by the film forming composition be resistant to water, for instance when immersed in water for an extended duration, and on the other hand, that the film be removable from the skin by cleaning with a wet wipe, without the use of aggressive organic solvents.

Film forming modified legume starches, and their uses in cosmetics or pharma, are known from the prior art.

In the field of cosmetics, specifically, from WO2020/128253, hydroxypropylated and hydrolyzed pea starch are known for their good film forming property and their good solubility in water. Films formed with these pea starches are easily removed from the skin by a gentle washing with still water. It is therefore obvious that such films could not be water resistant.

From WO2020/201071, the barrier properties and the fixative properties of hydrolyzed and hydroxypropylated pea starch are also known. From WO2022/199889, the styling properties on the hair are known.

Hydrophobic film forming modified starches known from the prior art are known from various technical fields. In the paper and board industry, film forming hydrophobic grafted starches are used to replace styrene-butadiene latexes in the coating of papers and boards.

### Technical Problem

In the field of water-resistant film former, well known and established polymers are synthetic ones such as styrene butadiene copolymers, polyvinylpyrrolidone polymers, and copolymers of vinylpyrrolidone and acrylates or methacrylates. Despite their great performances, these synthetic polymers have the big disadvantage of not meeting two of the main requests for nowadays consumer products: they are not natural or of natural origin (i.e. "naturality"), and they are not biodegradable.

In order to solve these two market needs, film formers of natural origin and biodegradable are already available, and improvements are still undergoing. For instance, such natural polymers are gums, such as pullulans, arabic gum, alginates, carrageenans; cellulosic materials and starches. Amongst starches, film forming and water resistance properties are not easy to meet at the same time.

Starches have been known for a long time for their film forming properties.

The most well-known use of this property of starches is the stiffening of clothes, which dates to antiquity. It relies on the ability of a starch suspension to form a film at the surface of the fibers by drying. The starch films are somehow very soluble in water and has low water resistance.

Another well-known and more recent use of this film forming property is the coating of papers, where hydrolyzed starches form a smooth continuous film at the surface of the paper and determine the printing performances through its affinity with inks. All usual native starches may be used, but the preferred botanical origin for this starch is waxy starch, which is a starch with an amylopectin content greater than 99 wt%. it is due to its easiness of implementation, specifically its easiness of cooking and stability after cooking, and to the excellent film forming property of amylopectin. Those waxy starch films have a very high affinity with water, which is looked for to make the paper printable, but which also is a big drawback when resistance to water is needed.

To increase the resistance to water of films formed based on waxy starch, the addition of synthetic polymers is mandatory. For instance, synthetic polymers of the styrene butadiene latex type are usually added in the starch-based coating solution of paper, which brings back the issue of lack of biodegradability and naturality.

Another means of obtaining a water-resistant film based on starch consist in functionalizing common starch (wheat, corn) with hydrophobic groups, such as esterification with fatty acids, or transesterification with anhydrides of fatty acids, for instance with the commonly and widely used alkenyl succinate anhydrides, or such as etherification with fatty alcohols. Common sodium and calcium salts of these starches are very soluble in water, and do not yield films that are enough resistant to water. Their highly functionalized alternatives, with degree of substitution higher than 1 or 2, lost their naturality and biodegradability. Only the aluminum salts of such alkenyl succinate starches in granular state are hydrophobic enough to be water repellant. Somehow, it is very difficult to form films with those granular hydrophobically functionalized starches, because of their very poor film forming intrinsic property, which makes them not adapted to form a film on a surface, for instance on the skin.

Still another means to obtain a water-resistant film is to use starches rich in amylose. Native high amylose starches are very difficult to use because they require to be cooked at above 80°C and will rapidly form gels when cooled to room temperature, which are prone to phase separation known as syneresis. This instability renders the formulated product instable during storage over time. These native high amylose starches are not usable. They must be modified to be stable enough.

As an example, Roquette has developed as a film forming starches with a high index of natural origin, noted INO, the pregelatinized hydrolyzed hydroxypropylated pea starch sold as "Beauté by Roquette ST 720" by Roquette has a INO of 0.93. This starch is stable thanks to the hydroxypropylation and is very easy to use and to introduce into formulated products. This film former is very effective at forming non occlusive films on the skin or the hair, that protect these from external aggressions (patcit1) and that also have fixative effect for make-up

(patcit1) and styling effects for the hair (patcit2). This kind of starch is also easily solubilized in water, which can be an advantage for easy removal by washing, but which can also be a disadvantage, for instance, when it bleaches from its support when exposed to moisture or immersed in water. This starch does not allow the formation of water resistant or waterproof films.

The applicant continued its research to obtain a new starch which possess all the below required properties:

- have a high index of natural origin (INO) according to the ISO 16128-2:2017,
- be easy to use, specifically easy to disperse or incorporate into water or oil,
- be a film former that form water-resistant films when used alone, or waterproof films when used in combination with other hydrophobic materials,
- be stable when used in formulated products, which means specifically that it does not generate any phase separation or syneresis, and which means a chemical and physical compatibility with other classical ingredients used in the formulated products,
- form a washable film, easy to remove by rubbing with water or wiping with a fabric or cellulosic material,
- be biodegradable according to OECD method chosen from 301B, 301D or 301F.

In a surprising and unexpected way, the present invention meets all these previous properties. The applicant has found that a physical process, known but still difficult to master, namely pregelatinization, particularly on a drum dryer, can be used to obtain a starch with those properties. This results in increased resistance to dissolution in water for films formed from aqueous formulations containing the said starch. This is due to the previously unknown specific selection of operating conditions for such a physical process. This starch allows a good balance between the starch's ability to dissolve into water, and its ability to resist dissolution in water.

## Brief Description of Drawings

Other features, details and advantages will be shown in the following detailed description and on the figures, on which:

### Fig. 1

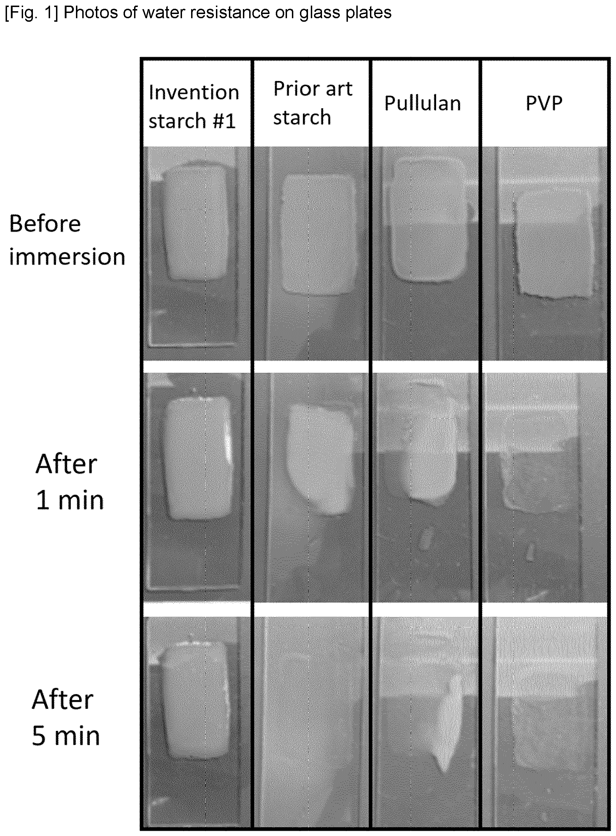

[Fig. 1] shows the water resistance according to the "glass sheet test" for a modified legume starch according to the present invention, compared with a commercial modified legume starch Beauté by Roquette® ST 720, and with a hydrophobic film-forming synthetic polymer, Polyvinylpyrrolidone.

### Fig. 2

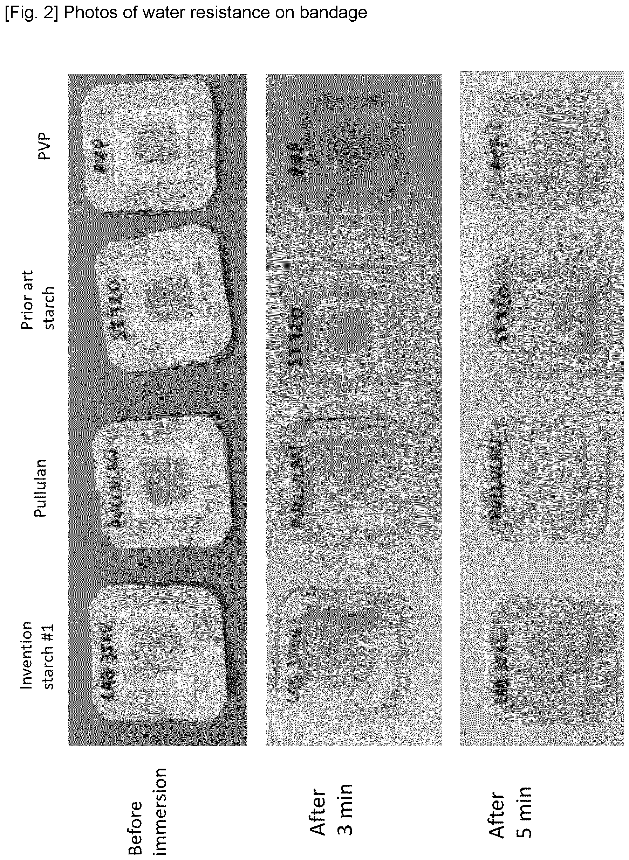

[Fig. 2] shows the water resistance according to the "bandage test" for a modified legume starch according to the present invention, compared with a commercial modified legume starch Beauté by Roquette® ST 720, and with a hydrophobic film-forming synthetic polymer, Polyvinylpyrrolidone.

### Fig. 3

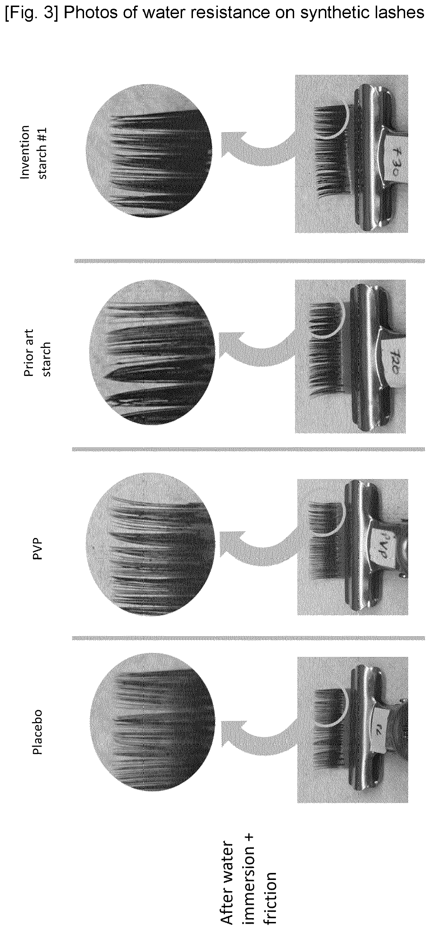

[Fig. 3] shows the water resistance according to the "mascara test" for a modified legume starch according to the present invention, compared with a hydrophobic film-forming synthetic polymer, Polyvinylpyrrolidone.

## Description of Embodiments

Embodiment 1: pregelatinized hydrophilically modified legume starch having a RVA viscosity profile characterized by a final cold viscosity from 100 to 2000 cPo, or 125 to 1750 cPo, or 150 to 1500 cPo, or to 200 to 1200 cPo, or from 600 to 1000 cPo, or from 650 to 950 cPo, the RVA temperature and stirring profile being the following:

- at time= 0 s: temperature= 25°C; stirring speed= 100 rpm,
- at time= 10 s: temperature= 25°C; stirring speed= 500 rpm,
- at time= 30 s: temperature= 25°C; stirring speed= 160 rpm,
- at time= 10 min: temperature= 25°C; stirring speed= 160 rpm,
- at time= 18 min: temperature= 90°C; stirring speed= 160 rpm,
- at time= 21 min: temperature= 90°C; stirring speed= 160 rpm,
- at time=29 min: temperature= 30°C; stirring speed= 160 rpm,
- at time=34 min: temperature= 30°C; stirring speed= 160 rpm.

Embodiment 2: starch according to embodiment 1, wherein its RVA viscosity profile is characterized by an initial cold viscosity from 250 to 4000 cPo, or from 400 to 3500 cPo, or from 500 to 3000 cPo.

Embodiment 3: starch according to embodiment 1 or 2, wherein its RVA viscosity profile is characterized by a hot viscosity from 50 to 900 cPo, or from 75 to 800 cPo, or from 100 to 700 cPo.

Embodiment 4: starch according to any of embodiments 1 to 3, wherein the RVA viscosity profile is characterized by:

- an initial increase of the viscosity at a rate of at least 1500 cPo/min,
- a decrease of the viscosity during the heating step at a rate of at least 100 cPo/min,
- an increase of the viscosity during the cooling step at a rate of at least 25 cPo/min.

Embodiment 5: starch according to any of embodiments 1 to 4, wherein the legume starch has an amylose content greater than or equal to 20 wt%, or 24 wt%, or 30 wt%, or 34 wt%, preferentially from 30 to 40 wt%.

Embodiment 6: starch according to embodiment 5, wherein the legume starch is chosen from pea starch, bean starch, fava bean starch, faba bean starch, lentil starch, alfalfa starch, clover starch, lupin starch, or soybean starch, preferentially is a pea starch.

Embodiment 7: starch according to any of embodiments 1 to 6, wherein the starch is hydroxyalkylated and/or carboxyalkylated, preferentially is hydroxyalkylated.

Embodiment 8: starch according to embodiment 7, wherein the starch is hydroxypropylated.

Embodiment 9: starch according to any of embodiments 1 to 8, wherein the degree of substitution is from 0,01 to 3, or from 0,05 to 0,8, or from 0,05 to 0,6, or from 0,05 to 0,21, preferentially 0,15 to 0,19.

Embodiment 10: starch according to any of embodiments 1 to 9, wherein the starch is not hydrolyzed, preferentially not hydrolyzed by chemical or enzymatic means.

Embodiment 11: starch according to any of embodiments 1 to 10, wherein the starch has a solubility in water at 22°C from 25 to 80 %, or from 30 to 75%, or from 35 to 60%, or from 45 to 55%.

Embodiment 12: starch according to any of embodiments 1 to 11, wherein the particle size distribution of the starch has a volume-average mean diameter D(4,3) from 25 to 200 microns, or from 40 to 150 microns, or from 50 to 100 microns.

Embodiment 13: starch according to any embodiments 1 to 12, having a hydrophobicity value as measured according to the model of Young-Laplace and Fowkes equal to or greater than 30%, or 35%, preferentially from 30 to 55%, or from 35 to 50%.

Embodiment 14: process of manufacturing of a pregelatinized hydrophilically modified legume starch as described in any of embodiments 1 to 13, comprising, preferentially consisting of, the steps of:

   a) providing a native legume starch,
   b) hydrophilically modifying, preferentially hydroxypropylating, the native legume starch of step a,
   c) pregelatinizing the hydrophilically modified legume starch of step b,
   d) grinding the pregelatinized hydrophilically modified legume starch of step c).

Embodiment 15: process according to embodiment 14, wherein the pregelatinizing step is performed using a drum dryer or an extruder, or a combination of a jet cooker and a spray-dryer, preferentially is performed using a drum dryer.

Embodiment 16: process according to any of the any of embodiments 14 or 15, wherein there is no step of hydrolysis of the starch by a chemical or enzymatic means.

Embodiment 17: use of a pregelatinized hydrophilically modified legume starch as described in any of embodiments 1 to 13, in a formulated product chosen from cosmetic product, a pharmaceutical product, a dermatologic product, a veterinarian product, an industrial product, a plant care product, or a food product.

Embodiment 18: use according to embodiment 17, wherein the starch is a water-resistant film former.

Embodiment 19: use according to any of embodiments 17 or 18 to increase the water resistance of a film formed on a surface by the formulated product, preferentially on a human skin or a human hair.

Embodiment 20: use according to any of embodiments 17 to 19, wherein the cosmetic product is chosen from a skin care product, a hair care product, a make-up product, an oral care product, or a hygiene product.

Embodiment 21: use according to embodiment 20, wherein the skin care product is chosen from sun care products.

Embodiment 22: use according to embodiment 20 wherein the hygiene product is a soap, a deodorant.

Embodiment 23: use according to embodiment 20, wherein the make-up product is chosen from mascara, eye shadow, eyeliner, foundation, tattoo make-up, nail varnish.

Embodiment 24: film forming composition comprising a pregelatinized hydrophilically modified legume starch as described in any of embodiments 1 to 13, in a physiologically acceptable medium.

Embodiment 25: film forming composition according to embodiment 24, wherein the content in the pregelatinized hydrophilically modified legume starch is from 0,5 to 10 weight % of the total weight of the film forming composition, or from 3 to 7 weight %, or from 4 to 6 weight %.

Embodiment 26: film forming composition according to any of embodiments 24 or 25 comprising at least one other starch chosen from native granular starch, native cooked starch, a modified starch.

Embodiment 27: film forming composition according to embodiment 26, wherein the modified starch is chosen from hydroxyalkylated and hydrolyzed starches.

Embodiment 28: film forming composition according to any of embodiments 24 to 27, comprising a plasticizer, preferentially chosen from polyols, urea, organic acids and their salts, betaine, or combination thereof, preferentially chosen from glycols, glycerol, sorbitol, xylitol, mannitol, isosorbide.

Embodiment 29: film forming composition according to any of embodiments 24 to 28, comprising an alcohol, preferentially ethanol.

Embodiment 30: film forming composition according to any of embodiments 24 to 29, comprising:

- a film forming polymer chosen from synthetic polymers, semi-synthetic polymers, and natural polymers,
- and/or a thickening or gelling agent.

Embodiment 31: film formed on the surface of a human or animal skin, mucous membrane, or hair, comprising, or consisting of, a pregelatinized hydrophilically modified legume starch as described in any of the embodiments 1 to 13, or obtained by the application of a film forming composition as described in any of embodiments 24 to 30.

Embodiment 32: film according to embodiment 31, having a resistance to water equal to or higher than 1 minute according to test A, or equal to or higher than 2 minutes, or equal to or higher than 3 minutes, or equal to or higher than 4 minutes, or equal to or higher than 5 minutes.

Embodiment 33: film according to any of embodiments 31 or 32, having a contact angle with a drop of water as measured by goniometry, equal to or higher than 70° after 1 minute of contact between the drop and the film, and/or 60° after 5 minutes of contact between the drop and the film.

Embodiment 34: make-up product comprising a pregelatinized hydrophilically modified legume starch as described in any of embodiments 1 to 13, forming a film as described in any of embodiments 31 to 33.

Embodiment 35: sun care product comprising a pregelatinized hydrophilically modified legume starch as described in any of the embodiments 1 to 13, forming a film as described in any of embodiments 31 to 33

Figures and the following detailed description contain, essentially, some exact elements. They can be used to enhance understanding the invention and, also, to define the invention if necessary.

The starch according to the invention, referred herein as "the starch", is a pregelatinized hydrophilically modified legume starch, which may be characterized by specific values of viscosities using a viscosity measurement called "RVA". Each of these characteristics of the starch are described hereafter.

**Legume starch:**

The term "legume starch" refers to any composition extracted from a legume, particularly a legume belonging to the family of papilionaceae, and whose starch content is greater than 40%, preferably greater than 50%, and even more preferably greater than 75%, with these percentages being expressed on a dry weight basis relative to the dry weight of said composition. Advantageously, this starch content is greater than 90% (dry/dry). It can be particularly greater than 95%, including greater than 98%.

The term "legume" as used in the present application refers to any plant belonging to the families of cesalpiniaceae, mimosaceae, or papilionaceae, and particularly any plant belonging to the family of papilionaceae, such as, for example, peas, beans, fava beans, faba beans, lentils, alfalfa, clover, lupins, or soybeans. This definition notably includes all plants described in any of the tables contained in the article by R. HOOVER et al. entitled "Composition, structure, functionality and chemical modification of legume starches: a review" (Can. J. Physiol. Pharmacol. 1991, 69 pp. 79-92).

Preferably, the legume is chosen from the group comprising peas, beans, fava beans, and faba beans.

Advantageously, it is peas, with the term "peas" being considered here in its broadest sense and including in particular:

- all wild varieties of "smooth pea", and
- all mutant varieties of "smooth pea" and "wrinkled pea", regardless of the uses to which said varieties are generally intended (human food, animal nutrition, and/or other uses).

Said mutant varieties notably include those denoted "mutants r", "mutants rb", "mutants rug 3", "mutants rug 4", "mutants rug 5", and "mutants lam" as described in the article by C-L HEYDLEY et al. entitled "Developing novel pea starches" Proceedings of the Symposium of the Industrial Biochemistry and Biotechnology Group of the Biochemical Society, 1996, pp. 77-87.

**Hydrophilically modified:**

The starch according to the invention is a hydrophilically modified legume starch. This means a legume starch that was modified by at least one chemical treatment that substituted a part of the hydroxyl groups of starch by other hydrophilic groups.

The chemical treatment consists in functionalizing the starch, that is to say in substituting the hydroxyl groups of anhydroglucose units by other chemical groups that are not naturally present in the starch, by etherification or by esterification of said hydroxyl groups. Such functionalization modifies the physicochemical properties of the starch.

The chemical modifications, also called "chemical functionalizations", useful for the present invention are the hydrophilic modifications, thus yielding a hydrophilically modified starch. In particular, these may be chosen from hydroxyalkylations, carboxymethylations, or combination thereof.

According to one embodiment of the present invention, the hydrophilic functionalization is a hydroxyalkylation, for instance the hydroxypropylation, which is also often referred to as a "stabilization" due to its beneficial effect on the retrogradation of a gel formed by the starch. Thus, according to one embodiment, the starch is hydroxypropylated.

"Hydroxypropylated" as used in the present invention refers to a starch substituted with hydroxypropyl groups by any technique known to those skilled in the art, for example by etherification reaction with propylene oxide, particularly having a hydroxypropyl group content characterized by the degree of substitution, which is from 0,01 to 3, or from 0,05 to 0,8, or from 0,05 to 0,6, or from 0,05 to 0,21, preferentially 0,15 to 0,19. This content may be determined in particular by proton nuclear magnetic resonance spectrometry, particularly according to the standard EN ISO 11543:2002 F.

**Not hydrophobically modified:**

The starch according to the present invention is not hydrophobically modified. This means that the starch was not chemically modified, or chemically functionalized, with any hydrophobic or amphiphilic groups, for instance such as alkenyl succinate groups.

**Very slightly hydrolyzed or not hydrolyzed:**

The starch according to the invention has a molecular weight equal to the one of native starch, or slightly lower than the one of native starch.

**Pregelatinized:**

A pregelatinized starch is a dry powder of partially or totally gelatinized starch. It is usually obtained by submitting the starch to a gelatinization and a simultaneous or subsequent drying.

The gelatinization is a physical treatment that consists in altering the physical structure of the starch granules by submitting the starch to a thermal and/or mechanical process. Gelatinization is the transformation of the starch granules into a starch paste, also called starch glue due to its gluing property. By the action of temperature in suspension in water which triggers the swelling of starch granules by water absorption, the granules are almost totally disrupted, thereby releasing their content into the aqueous phase, and thus forming a colloidal suspension. The suspension of starch obtained by gelatinization is called a gelatinized starch and may further be transformed into a powder by drying, which yields a powder readily dispersible into water without any cooking, and sometimes even without any heating, for instance in cold water. The powder of gelatinized starch is called a pregelatinized starch. The process of making a pregelatinizes starch is called pregelatinization.

## Pregelatinization

Pregelatinization may be carried out in a single process step that performs both gelatinization and drying, or by carrying out several process steps placed in series, usually a first gelatinization step followed by a second drying step. It may be carried out on any technical equipment able to heat an aqueous suspension of starch at a temperature above the gelatinization temperature of starch, usually above 70°C, while also being able to handle the high viscosity gelatinized starch thus obtained, and also able to dry the gelatinized starch generated.

Gelatinization may be carried out in a stirred tank or in a jet cooker. Pregelatinization may be carried out in a drum drier, or in an extruder. Another pregelatinization process may be the spray drying of a gelatinized starch obtained by jet cooking for instance.

According to one embodiment, the starch is pregelatinized on a drum dryer.

This typical equipment used for gelatinizing starch and at the same time drying the obtained gelatinized starch, called the drum dryer, also called drum cooker, is presented in the chapter XXII, p.523-536, written by Eugene L. Powell in "Starch: Chemistry and Technology, volume II Industrial aspects" by Whistler and Paschall, 1967. A drum dryer may comprise one heated revolving drum (single-drum dryer) or two heated drums rotating towards each other (double-drum dryer) or rotating away from each other (twin-drum dryer). The drum dryer transforms an aqueous suspension of starch into a powder of dried gelatinized starch, called pregelatinized starch.

According to one embodiment of the process useful for obtaining the modified legume starch according to the invention, the pregelatinization is carried out:

- on a suspension of native granular starch in water at a concentration from 5 to 50 weight%, or from 10 to 40 weight%, or from 20 to 35 weight% with respect to the total weight of the aqueous starch suspension, also called a "starch milk" due to its milky texture,
- at a temperature above 70°C, or 80°C or 90°C, said temperature being either the temperature set for the heating surfaces or for the starch suspension,
- for a duration of at least 1 second.

## Grinding:

The pregelatinized starch may be grinded into a powder by a grinding step, that shape the pregelatinized starch into particles and that control the granulometry of these particles.

When the pregelatinization is carried out on a drum dryer, the film of pregelatinized starch recovered at the scraping blade, is sent into a grinder, to transform the film into particles. The grinded pregelatinized starch can be packaged, and can be appropriately used, for instance added into formulated products.

According to one embodiment, the starch according to the invention is grinded in a hammer crusher, also called hammer mill, or in a percussion and attrition grinder, or in a combination thereof.

## RVA viscosity profile:

Without being bound to a theory, the applicant believes that the specific modified legume starch according to the invention undergoes a reorganization of its constitutive macromolecules during its retrogradation, that allows the formation of a film that exhibits an unprecedented hydrophobicity for a film formed by a non-hydrophobically modified legume starch.

An analytical method of choice for characterizing the retrogradation of starches is the measurement of the viscosity profile using a Rapid Visco Analyzer. This measurement consists in measuring the viscosity of an aqueous suspension of starch during a controlled program of heating and cooling, wherein the mixer serves as a viscosimeter.

The modified legume starch according to the invention is characterized:

- during the first stage of steady heating at 25°C: by an initial increase of the viscosity at a rate of at least 1500 cPo / min; and by a plateau of viscosity of at least 2000 cPo,
- during the second stage of constant increase of heating from 25°C to 90°C in 8 minutes: by a decrease of its viscosity to a value ranging from 250 cPo to 750 cPo, named "hot viscosity", at a rate of at least 100 cPo / min,
- during the third stage of constant decrease of temperature from 90°C to 30°C in 8 minutes: by an increase of viscosity to a viscosity at least 250 cPo higher than the hot viscosity, at a rate of at least 25 cPo / min,
- at the end of the third stage of steady heating at 30°C: by a viscosity of at least 500 cPo, or a viscosity from 500 to 1000 cPo.

**Process of production of a starch according to the invention:**

The modified legume starch according to the invention may be obtained by a process comprising or consisting of:

- a step a) of chemical functionalization by at least one of hydroxyalkylation and/or carboxyalkylation,
- a step b) of pregelatinization.

According to one embodiment of the process useful for obtaining a modified legume starch according to the present invention, the process comprises, or consists of:

- a first step a) of hydroxypropylation of a native pea starch in granular phase up to a degree of substitution of from 0,05 to 0,21, or up to 0,16 to 0,19,
- a second step b) of pregelatinization of the hydroxypropylated granular pea starch obtained in step a), on a drum dryer (also called drum cooker), at a starch content of 27 weight%, at a temperature of at least 90°C,
- a third step c) of grinding the film of pregelatinized hydroxypropylated starch from step b,
- a fourth step d) of collecting a powder of pregelatinized hydroxypropylated pea starch obtained in step b) and subsequent cooling at 20-25°C, which is called the pregelatinized hydroxypropylated starch.

Preferentially, the process does not comprise any steps of acidic or enzymatic hydrolysis of the starch, let it be the native granular pea starch, the hydroxypropylated granular pea starch or the pregelatinized hydroxypropylated pea starch.

**Water-resistant film former**

In the present application, "water-resistant film former" means a film former that is able to form a film that has water resistance, that is to say that is water-resistant. The film is usually formed by applying a formulated product comprising the starch according to the invention on a surface, and making or letting said formulated product dry.

**Water resistance:**

In the present application, "water resistance" means the ability to withstand the exposition to humidity and/or the immersion into water during several minutes or hours, without being immediately completely washed away by said humidity or water.

According to one embodiment, "water resistance" means the ability for a film formed at the surface of a glass plate to withstand immersion into agitated tap water at 21°C for at least 1 minute, or at least 2 minutes, or at least 3 minutes, or at least 4 minutes, or at least 5 minutes. The film is formed by spreading an aqueous solution of film former at 6 wt% prepared by dissolution of said film former at 21°C, on a glass plate, and letting it dry at 21°C for 12 hours.

**Other properties of the film formed with the starch according to the invention:**

In addition to being water resistant, the films formed by the starch according to the invention, or by formulated products comprising said starch, provides a tensing effect, and are elastic and not brittle.

**Applications:**

Thanks to its water-resistant film forming properties, the starch according to the invention may be used in various fields, such as pharmaceuticals, healthcare, food, personal care or cosmetics, home care and industrial applications. In cosmetics, applications are for instance in skin care, make-up and sun care. Examples of make-up formulations where the water-resistant film former starch according to the invention is useful are eyeshadow, eyeliner, mascara, foundation,

Examples of skin care formulations where the water-resistant film former starch according to the invention is useful are comforting creams or serums, eye serum, tightening serum, sun care cream.

**Cosmetic formulations:**

Co-film formers can be added to the starch according to the invention to improve the water resistance, such as co-polymers of vinylpyrrolidone and (meth)acrylates, like Antaron™ V-220F (Ganex™ V-220F) polymer, or such as co-polymers of vinylpyrrolidone and alkenes, like the VP/eicosane copolymer.

**Analytical methods:**

Viscosity profile by RVA measurement

RVA stands for Rapid Visco Analyzer. It is a physical measurement technic used for decades in the field of starches and modified starches. The evolution over time of the viscosity of a starch suspension under agitation subjected to a defined temperature profile is measured with a viscosimeter called RVA 4500 from Perkin Elmer. This evolution of the viscosity, aka the "RVA viscosity profile", is characteristic of the starch, and is peculiarly dependent on its botanical type, and the physical and chemical modifications the starch underwent.
The measurement protocol is as follows:

1) a weight of 1.7 g of dry starch and 4.5 g of glycerin are introduced directly into the bowl of the RVA viscosimeter, and homogenized using a spatula by manual mixing,
2) aside, prepare 21,8 g +/-0.01 g of water at room temperature,
3) prepare the RVA apparatus so that it displays "please press down tower" or a similar message,
4) pour the quantity of water prepared in step 2 in the starch/glycerin blend inside the bowl, and start the measurement immediately.

The starch suspension is subjected to the stirring and temperature profile according to table 1 and as visible on figure 1, and the viscosity is simultaneously measured by measurement of the torque applied on the stirring shaft.

[Table 1]

| Time (mm:ss) | Temperature (°C) | Stirring speed (rpm) | Identification of viscosity value |
|---|---|---|---|
| 0 | 25 | 100 | none |
| 00:10 | 25 | 500 | |
| 00:30 | 25 | 160 | Initial cold viscosity, also called "peak viscosity" |
| 10:00 | 25 | 160 | |
| 18:00 | 90 | 160 | Hot viscosity |
| 21:00 | 90 | 160 | |
| 29:00 | 30 | 160 | Final cold viscosity |
| 34:00 | 30 | 160 | |

The viscosity measured by the RVA viscosimeter is expressed in RVU, and is converted in centipoise, cPo, using the conversion: 1 RVU unit = 12 cPo. As a reminder, 1 cPo = 1 mPas.s.
The maximum of the viscosity occurring during the initial steady phase at 20°C from 30 sec to 10 min, is called the initial cold viscosity, also called "peak viscosity". The minimum of the viscosity occurring during the 90°C plateau from 18 min to 21 min, is called the hot viscosity. And finally, the maximum of viscosity occurring during the last steady phase at 30°C from 29 min to 34 min, is called the final cold viscosity.

Solubility in water at 22°C

The principle of this measure is the dispersion of a starch sample in water, followed by centrifugation to separate the solid and the liquid supernatant, and then analysis of the dry content of the supernatant. The protocol is the following:
Step 1) In a 250 ml beaker, add 200 ml of distilled water. Stir magnetically and add about 5 grams, accurately weighed using a 1/1000th of a gram precision balance and noted m_sample, of the sample to be analyzed. Homogenize for 15 minutes at 22°C using the magnetic stirrer. Centrifuge for 10 minutes at 4000 rpm.
Step 2) Take 25 ml of the supernatant liquid, introduce it into a tared crystallizer. Place in the oven at 60°C until the water evaporates. Then place in the oven at 103°C +/-2°C for 1 hour. Place into the desiccator and allow to cool to room temperature. Weigh the mass of dry residue, noted m_dryresidue, using a 1/1000th of a gram precision balance. The content of soluble starch, expressed as a percentage by mass, of the sample of starch, is given by the formula:

[Math. 1]

$$Solubility = \frac{m\_dryresidue \; x \; 200 \; x \; 100}{25 \; x \; m\_sample}$$

The solubility is thus expressed as percentage, which is the percentage of the mass of sample of starch that can dissolve into water.

Size distribution measurement by dry powder laser diffraction

The volume average diameter of the pregelatinized hydrophilically modified legume starch is measured by laser diffraction in dry powder.

Hydrophobicity value

The determination of the hydrophobicity value of the starch in an aqueous solution is based on the determination of the liquid-vapor surface tension, noted $\gamma_{LV}$, and their polar, $\gamma_{LV}^P$, and dispersive, $\gamma_{LV}^D$, components, of a liquid aqueous solution of starch in equilibrium with air.

In a first step, the liquid-vapor surface tension of the aqueous starch solution is measured by a force tensiometer, model K100C from KRÜSS. This gives the liquid-vapor surface tension $\gamma_{LV}$. The measurement is performed using a ring, and at room temperature thanks to a thermos regulated cell. The ring of defined geometry is cleaned with water and dried over a flame for a few seconds using a kitchen blowtorch. At least 50 mL of the liquid to be analyzed is placed into a glass crystallizer. Using the elevator, the liquid is raised and brought into contact with the ring which is suspended from a precision balance. Then the ring is submersed in the liquid. The force acting on the ring is measured until the value is stable, and then used to calculate the liquid-vapor surface tension $\gamma_{LV}$ in mN/m.

In a second step, the contact angle $\theta$ between the aqueous starch solution and a Teflon plate is measured by goniometer, model DSA100 from KRÜSS. Five microdrops of 3 microliters are placed on a Teflon plate. For each drop, the average of the angle is given. To respect the symmetry of the drop, each measurement giving a gap over 5 degrees between right and left angles is excluded. Contact angles are measured at the triple point, where there is solid, liquid and vapor.

Using the model of Young-Laplace and Fowkes according to the mathematical formula #2, the dispersive component of the solid-liquid surface tension $\gamma_{LV}^D$ of the solution of starch can be calculated:

[Math. 2]

$$(cos \, \theta + 1)^2 \, \gamma_{LV}^2 = 4(\gamma_{LV}^D . \gamma_{SV}^D)$$

with the value of the liquid-vapor surface tension $\gamma_{LV}$ as measured in the first step, and with the known value of the dispersive component of the solid-vapor surface tension Ysv° of Teflon, which is 22.4 mN/m.

And finally, the polar component of the liquid-vapor surface tension $\gamma_{LV}^P$ is calculated using the following relation:

[Math. 3]

$$\gamma_{LV} = \gamma_{LV}^D + \gamma_{LV}^P$$

The hydrophobicity value and the hydrophilicity value are then calculated according to the following formulas:

[Math. 4]

$$Hydrophobicity\ value\ (\%) = 100\frac{\gamma_{LV}^D}{\gamma_{LV}}$$

[Math. 5]

$$Hydrophilicity\ value\ (\%) = 100\frac{\gamma_{LV}^P}{\gamma_{LV}}$$

**Examples**

**Example 1: preparation modified legume starch according to the invention**

Pregelatinized hydroxypropylated pea starches according to the invention were prepared by first hydroxypropylating a native pea starch in granular phase, then simultaneously gelatinizing and drying the hydroxypropylated granular pea starch on a contact drum dryer, and finally grinding the pregelatinized hydroxypropylated pea starch to obtain a powder, which is usually called a pregelatinized starch, more precisely a pregelatinized hydroxypropylated pea starch in our case.

The native pea starch was obtained from Roquette Frères, France.

The hydroxypropylation was carried out on a starch suspension at 20-30 weight% in water with 0,5 M of sodium sulfate, by introducing propylene oxide in a stirred tank pressurized, using 0,5 M of sodium hydroxide as a catalyst, until the desired degree of substitution was reached. The hydroxypropylated starch was then recovered by filtration, washed with distilled water and dried to moisture content of about 12 weight% using a flash dryer.

A suspension of hydroxypropylated pea starch was then prepared by dispersing said starch into water at 27 wt% dry matter, under mixing and was then sent to the pregelatinization step. 10 ppm of Hydrogen peroxide was added to the starch suspension.

The pregelatinization was carried out on an indirectly heated contact single-drum dryer referenced as "model E 5/5" from the manufacturer ANDRITZ Gouda (documentation available online at nplcit2 https://www.andritz.com/products-en/separation/contact-dryers/gouda-drum-dryer), heated from the inside by pressurized steam and equipped with four top applicator rolls. The operating parameters were:

- the feed flowrate of the starch suspension: 55 L/hr,
- the rotation speed of the main drum: 3,5 rpm,
- the pressure of the steam inside the drum: 7 bars, which corresponds to steam temperature of 165°C,
- the thickness of the pregelatinized starch film: from 1,2 mm at the far left applicator roll (which is the applicator roll located on the side of the knife), to 0,7 mm at the far right applicator roll,
- the rotation speed of the applicator rolls: driven by mechanical coupling with the main drum,
- the temperature of the starch milk inlet: room temperature 20°C +/-5°C,
- the height of the blade / knife: lowest position possible.

The pregelatinized starch obtained at the outlet of the drum dryer by scraping by the knife, is then sent to two grinders placed in series:

- firstly, a hammer crusher, from FITZ MILL, model SPV DKAS012, equipped with a 1000 microns grid, operated at full speed which is 4600 rpm according to operating manual,
- secondly, an "Ultrafin" grinder from POITTEMILL, operated at 60% of its full speed.

The pregelatinized hydroxypropylated pea starch was then characterized by a measurement of its viscosity profile using a "Rapid Visco Analyzer" from Perkin Elmer, noted RVA, operated according to the conditions specified here above. The solubility in water at 22°C, and its D(10), D(90) and D(4,3) diameter were also measured. The results are in table 2.

[Table 2]

| | Invention starch #1 | Prior art starch (Beauté by Roquette® ST 720) |
|---|---|---|
| *RVA measurements* | | |
| Initial cold viscosity at 25°C (cPo) | 1615 | 42 |
| Hot viscosity at 90°C (cPo) | 324 | 30 |
| Final cold viscosity at 30°C (cPo) | 594 | 36 |
| *Others analytical parameters* | | |
| Solubility in 22°C water (%) | 50.1 | 98.4 |
| D(10) (microns) | 23.6 | 139.5 |
| D(90) microns | 175.1 | 467.8 |
| D(4,3) (microns) | 91.7 | 289 |

The analytical characteristics of a starch according to the prior art, the Beauté by Roquette® ST 720 manufactured by Roquette, hereafter BBR ST 720, were also measured according to the same analytical methods. The BBR ST 720 is hydrolyzed hydroxypropylated pregelatinized pea starch. It only differs from the invention starch #1 by the characteristic that it is hydrolyzed. The values of the characteristics of the BBR ST 720 are significantly different from the values of the characteristics of the invention starch #1, which proves that those two starches are totally different.
In the following examples, the starch obtained was then characterized and used in cosmetics formulation that showed improved resistance to water in comparison to a hydrolyzed starch and to film former benchmarks.

**Example 2: characterization of the hydrophobicity in aqueous solution by force tensiometry and goniometry**

The polar and the dispersive components of aqueous solutions of pregelatinized hydroxypropylated pea starches prepared according to example 1 were determined using a combination of two analytical methods. Aqueous solutions of hydroxypropylated hydrolyzed pregelatinized pea starch, the Beauté by Roquette® ST 720 and aqueous solutions of polyvinylpyrrolidone (hereafter PVP), were also characterized likewise. Each aqueous solution was prepared by dissolution of a starch in water at 20°C under agitation. Table 3 presents the aqueous solutions characterized.

[Table 3]

| Aqueous Solution # | Composition |
|---|---|
| 1 | 6 wt% of BBR ST 720, adjusted at pH 4 |
| 2 | 6 wt% of PVP at pH 4 |
| 3 | 6 wt% of Starch #1 according to the invention at pH 4 |
| 4 | 6 wt% of BBR ST 720, adjusted at pH 7 |
| 5 | 6 wt% of PVP at pH 7 |
| 6 | 6 wt% of Starch #1 according to the invention at pH 7 |

As detailed in the description of the method for determining the hydrophobicity value, the polar and dispersive components of the aqueous starch solution were calculated from its liquid-vapor surface tension, the Teflon surface tension and the contact angle using the Young-Laplace and Fowkes model. Results are presented in table 4.

[Table 4]

| Solution # | Liquid-vapor interfacial tension (mN/m) | Polar component of liquid tension (mN/m) | Dispersible component of liquid tension (mN/m) | % of hydrophobicity | % of polarity |
|---|---|---|---|---|---|
| 1 | 50.4 | 34.3 | 16.1 | 32 | 68 |
| 2 | 59.5 | 34.1 | 25.4 | 43 | 57 |

(continued)

| Solution # | Liquid-vapor interfacial tension (mN/m) | Polar component of liquid tension (mN/m) | Dispersible component of liquid tension (mN/m) | % of hydrophobicity | % of polarity |
|---|---|---|---|---|---|
| 3 | 51.8 | 30 | 21.8 | 42 | 58 |
| 4 | 51.6 | 36 | 15.6 | 30 | 70 |
| 5 | 58 | 36.7 | 21.3 | 37 | 63 |
| 6 | 56 | 36.1 | 19.9 | 36 | 64 |

Aqueous solutions containing the hydroxypropylated pregelatinized pea starch according to the invention were significantly more hydrophobic than aqueous solutions containing the hydroxypropylated hydrolyzed pregelatinized pea starch BBR ST 720, both at pH 4 and pH 7. The level of hydrophobicity thus reached is equivalent to the one of aqueous solutions of polyvinylpyrrolidone.

**Example 3: characterization of the hydrophobicity of strips of films by goniometry (Test B)**

The contact angle of drops of water deposited on the surface of strips of film made with film formers chosen from the prior art starch, the invention starch #1 and the benchmark film former, were monitored at 1 minute and 5 minutes after deposition, with a goniometer KRÜSS DSA 100.

The strips were prepared as follows:

1) an aqueous solution of film former was prepared by dissolution of the film former in distilled water at room temperature at the appropriate content in film former, and then debubbled under vacuum during 5 minutes or until no bubble appeared anymore,
2) 4.5 g of the aqueous solution of film former was poured into a 7x1x1 cm silicone mould,
3) the filled mould was placed in a thermostated and humidity regulated oven, at 23°C and 50% relative humidity, during 72 h, in order to dry,
4) a rectangular strip of film was thus obtained, ready to be used.

The variation of the contact angle of the drop of water with the film at 1 and 5 minutes with respect to the initial contact angle (which means just after the deposition of the drop) were thus calculated and are presented in table 5.

[Table 5]

| Relative variation of contact angle with respect to initial contact angle | After 1 min | After 5 min |
|---|---|---|
| 6.7 wt% of BBR ST 720 at pH 4 | -30% | -54% |
| 6.7 wt% of starch #1 at pH 4 | -8% | -15% |
| 6.7 wt% of PVP at pH 4 | -69% | -85% |

Those variations are negative because of the affinity of water with the hydrophilic polymers constituting the films. The film former tested led to different momentum of this relative variation. The film made with the PVP had the biggest variations, which indicates that the drop of water spread on this film at the highest rate amongst the films tested. The film strip made with the prior art starch had a lower variation, which was somehow bigger that the variation measured on the film made with the invention starch #1. This film made with invention starch #1 had the lowest variations. This indicates that the drop of water spread much slower on the film made the invention starch #1 compared to the film made with the prior art starch and the benchmark. This means that the invention starch #1 made a film that is more resistant to water.

**Example 4: water resistance of films on surfaces (Test A)**

The dissolution in water of films formed on inert surfaces was visually evaluated for films formed with a starch according to the invention (starch #1 from example 1), a starch according to the prior art (BBR ST 720), and two film-former benchmarks (PVP and Pullulan), for two types of surfaces: a glass plate, and a bandage. For each surface and each film-former, the protocol was as follows:

1) prepare the aqueous solution with pigments,

2) spread 0.3 g on the surface, either glass plate or bandage, placed horizontally on a flat support, using a spatula,

3) dry at 22°C +/-3°C and 55 %relative humidity +/-5 for 12 hours, horizontally on a flat support,

4) Immerge in a beaker filled with water on a magnetic heater and stirrer, with stirring at 600 rpm for glass plates, and 1000 rpm for bandages, maintained at 21°C,

5) take pictures of the surface out of water at 1 min and 5 min for glass plates, and at 3 min and 5 min for bandages.

Results obtained on glass plates are presented on figure 2 and described in table 6. The film formed by the starch according to the invention remained unchanged after 1 min of immersion in water, whereas the prior-art starch and the benchmark film former largely disappeared. After 5 minutes of immersion, there remained about half of the film for the invention starch, whereas the two other films almost totally disappeared. These results showed the greater resistance to water dissolution of the film formed with the invention. Roughly, the water resistance of the film formed on glass plates with the invention starch is 2.5 times higher than the water resistance of the comparative films (prior art and benchmark).

[Table 6]

| Duration of immersion | 0 min | 1 min | 5 min |
|---|---|---|---|
| Prior-art starch | Homogenous and opaque yellow rectangular film | The corners started to disappear. The remaining film looked homogenous and opaque | The whole film disappeared |
| Invention starch | Homogenous and opaque yellow rectangular film | Homogenous and opaque yellow rectangular film | Homogenous and opaque yellow rectangular film |
| PVP | Homogenous and opaque yellow rectangular film | Film almost completely disappeared; only a light-yellow shade remained | Film almost completely disappeared; only a light-yellow shade remained |
| Pullulan | Homogenous and opaque yellow rectangular film | About one third of the film disappeared | About three quarters of the film disappeared |

Results obtained on bandages are presented on figure 3 and described in table 7. The film formed with the invention starch was able to withstand the immersion in water for a duration of 5 minutes, whereas the films formed the prior art starch of the benchmark film former did not withstand such an immersion, as they almost totally disappeared from the bandage.

[Table 7]

| Duration of immersion | 0 min | 3 min | 5 min |
|---|---|---|---|
| Prior-art starch | Homogenous film with medium yellow color, and a square shape, the fabric of the bandage is not visible | The film started to dissolve: the fabric of the bandage became visible; the angles of the square disappeared; half the quantity of matter remained visually | The film largely disappeared. Only a rounded thin layer remained |
| Invention starch | Homogenous film with medium yellow color, and a square shape, the fabric of the bandage is not visible | The film did not dissolve. It remained square and homogenous, with no visible loss of matter. It appeared with a lighter yellow color. | The film remained, homogenous, only slightly dissolved, keeping a visible square shape. |
| PVP | Homogenous film with medium yellow color, and a square shape, the fabric of the bandage is not visible | The film largely disappeared. Only a rounded thin layer remained, representing about half the quantity of matter of the prior-art starch film | The film almost totally disappeared, leaving a slight yellow shade, barely visible |

(continued)

| Duration of immersion | 0 min | 3 min | 5 min |
|---|---|---|---|
| Pullulan | Homogenous film with medium yellow color, and a square shape, the fabric of the bandage is not visible | The film started to dissolve: the fabric of the bandage became visible; the angles of the square disappeared; half the quantity of matter remained visually | The film largely disappeared. Only a thin layer remained |

The invention starch formed films with significantly higher resistance to water than the starch from the prior art and the film former benchmarks.

**Example 5: water resistance on fibers**

Mascaras prepared according to formula of table 8 comprising 5 wt% of one film former chosen from the starch of the prior art, the starch #1 according to the invention, the PVP, the pullulan, or not comprising any film former (placebo formula) were applied on synthetic, pink-colored lashes. The resistance of the mascara to immersion in water, and to further friction with a dry paper, was visually assessed for each film former.

[Table 8] mascara formulation for synthetic lashes water resistance test

| Phase | Ingredient; supplier | INCI | Weight % |
|---|---|---|---|
| A1 | Demineralized water | Aqua | 71.8 |
| A2 | Beauté by Roquette® DS 112 | Starch Acetate (and) Hydroxyethyl Cellulose (and) Xanthan Gum | 1 |
| A3 | Film former or nothing (placebo) | Not applicable | 5 or 0 (placebo) |
| A4 | Unipure Black LC 998 PHY | CI 77499 (and) Phytic Acid (and) Sodium Hydroxide | 10 |
| B1 | Cera Alba | Beeswax | 2 |
| | Copernicia Cerifera (Carnauba) Wax | Carnauba wax | 1 |
| | Candelilla Cera | Candelilla wax | 2 |
| | Ricinus Communis (Castor) Seed Oil | Huile de Ricin | 2 |
| | Cutina FS 45 | Stearic acid | 3.5 |
| B2 | Sodium Hydroxide | Soude 40% | 0.7 |
| C | Microcare PHC | Phenoxyethanol (and) Chlorphenesin (and) Glycerin | 1 |
| D | citric acid aqueous solution 10 wt% | Citric acid | |

Each mascara was prepared as follows:

1/ Weigh and mix phase A1,

2/ Add phase A2 and heat at 75-80°C for 25 min,

3/ Add A3 and homogenize for 15 MIN at 2200 rpm at 80°C and add pigments,

4/ Weigh and heat phase B1 at 80°C,

5/ Emulsify B1 in A (1-2min),

6/ Add B2, and let stir for 15 min at 2500 rpm at 80°C,

7/ Cool down very low agitation (1100 rpm) and at RT (no ice),

8/ Add C and adjust pH around 7.3

Each mascara were applied on one strip of synthetic pink-colored lashes as follows:

1/ Apply 0.04g of mascara on the set of lashes,

2 / Let dry 1 hour at 22°C +/-3°C,

3/ Take photo, referred as "before immersion into water"

3/ Immerse lashes in water at 21°C during 30s without movement,

4/ Stir lashes in said water 30s,

5/ Plunges in and out of the water several times during 30s,

6/ Stir lashes in said water 30s,

7/ Take photo, referred as "after immersion into water"

8/ Let dry at 22°C +/-3°C and rub the eyelashes back and forth on a tissue,

9/ Take photo, referred as "after drying and friction with tissue".

The photos taken for each mascara are presented on figure 3, and they are described in the following table 9.

[Table 9]

| Photography taken | Before immersion into water | After immersion into water | After drying and friction with tissue |
|---|---|---|---|
| Placebo | The lashes were homogeneously covered with mascara. The pink color was not visible. | There was visibly less mascara on the lashes. The pink color became slightly visible. | There was visibly significantly less mascara on the lashes. The pink color of the lashes was visible. |
| PVP | The lashes were homogeneously covered with mascara. The pink color was not visible. | There was visibly less mascara on the lashes. The pink color became slightly visible. | There was visibly significantly less mascara on the lashes. The pink color of the lashes was visible. |
| Prior art starch | The lashes were homogeneously covered with mascara. The pink color was not visible. | There was visibly less mascara on the lashes. The pink color became slightly visible. | There was visibly less mascara on the lashes. The pink color of the lashes was slightly visible. |
| Invention starch #1 | The lashes were homogeneously covered with mascara. The pink color was not visible. | There was visibly as much mascara on the lashes as before immersion into water. The pink color of the lashes was not visible. | There was visibly as much mascara on the lashes as before immersion into water. The pink color of the lashes was not visible. |

The lashes coated with the mascara comprising the prior art starch showed a visible loss of mascara after immersion into water, which was really significant after friction with a tissue, whereas no loss of mascara was observed for the lashes coated with the mascara comprising the invention starch #1. The invention starch #1 gave a mascara with a higher water resistance than the starch according to the prior art.

**Example 6: application in cosmetic: skin care and make-up formulations**

The invention starch was used as a film former providing water resistance in several cosmetic formulated products.

Eyeshadow formulation #1:

This formulation was prepared according to the composition of table 10 following the operating protocol below:

1) Prepare pre-mix phase A1 and heat up to 80°C,
2) Add phase A2 into phase A1 while stirring at medium speed, and maintain agitation and temperature 80°C for 40 minutes,
3) Heat phase B at 80°C and add to phase A1 +A2 under medium to strong agitation speed, and maintain agitation and temperature for 15 minutes,
4) Allow to cool down to room temperature while stirring gently.

[Table 10] eye-shadow formulation

| Phase | Ingredient; supplier | INCI | Weight % |
|---|---|---|---|
| A1 | Demineralized water | Aqua | 66.38 |
| | Microcare PHC; Thor | Phenoxyethanol, Glycerin | 1 |
| | Glycerin; Lubrisolve | Glycerin | 1 |
| | Covarine red WN 3798; Sensient | CI 7749, Glycerin, Xanthan gum, Sodium citrate, Aqua | 0.07 |
| | Covarine black WN 9798; Sensient | CI 77499, Glycerin, Xanthan gum, Sodium citrate, Aqua | 0.55 |
| | Invention starch #1 | Not available | 5 |
| A2 | Beauté by Roquette® DS 112 ; Roquette | Starch acetate, Hydroxyethyl cellulose, Xanthan gum | 3 |
| B | Imwitor 372P ; IOI Oleochemicals | Glyceryl stearate citrate | 1.1 |
| | Imwitor 900K ; IOI Oleochemicals | Glyceryl stearate | 1.9 |
| | Titanium dioxyde ; Cooper | Titanium dioxyde | 1.5 |
| | huile de ricin ; Cooper | Ricinus communis seed oil | 5 |
| | Labrafac CC ; Gattefossé | Caprylic/capric triglycerid | 5 |
| | Covapearl star doré 2375 ; Sensient | CI 77891, Syntethic fluorphlogopite | 2 |
| | Covapearl light brown 830 ; Sensient | CI 77491, Mica | 6.5 |

Eyeshadow formulation #2:

This formulation was prepared according to the composition of table 11 following the operating protocol below:

1) at room temperature, disperse the invention starch #1 in water while stirring with defloculator at 1000 rpm until homogenous dispersion is obtained,

2) add the xanthan gum to phase A while stirring until a homogenous mixture is obtained, taking care that there is no undissolved particle left,

3) add Beauté by Roquette ST 118 while stirring at 2000 rpm for 10 minutes minimum with a defloculator to form a gel,

4) add the ethanol and the Microcare PHC while stirring,

5) disperse the pearlescent pigment under slow stirring until a homogenous color is obtained,

6) adjust pH to 6.5

[Table 11] eye-shadow formulation

| Phase | Ingredient; supplier | INCI | Weight % |
|---|---|---|---|
| A | Demineralized water | Aqua | 76.2 |
| | Invention starch #1 | Not available | 5 |
| B | Xanthan Gum; Jungbunzlauer | Xanthan Gum | 0.3 |
| C | Beauté by Roquette® ST 118; Roquette | Sodium Carboxymethyl Starch | 5 |
| D | Microcare PHC; Thor | Phenoxyethanol, Chlorphenesin, Aqua, Glycerin | 1 |
| | Absolute Ethyl Alcohol; Cooper | Ethanol | 5 |
| E | Covapearl Light Brown 830; Sensient | Mica, Cl 77491 | 7.5 |

Skin care formulation #1:

This formulation was prepared according to the composition of table 12 following the operating protocol below:

1) Mix and heat the constituents of phase A to 85°C while stirring at 1600 rpm with a defloculator,

2) Cool phase A to 35°C and add phase C,

3) Homogenize phase B aside, and then add to phase A+C under strong stirring to emulsify,

4) adjust to pH 6 with phase D if necessary.

[Table 12] conforting serum

| Phase | Ingredient; supplier | INCI | Weight % |
|---|---|---|---|
| A | Demineralized water | Aqua | 75.25 |
| | Beauté by Roquette® ST 320 | Hydroxypropyl Starch Phosphate | 5 |
| | Beauté by Roquette® PO 500 | Isosorbide | 3 |
| | Invention starch #1 | Not available | 6 |
| B | Eumulgin SG | Sodium Stearoyl Glutamate | 0.2 |
| | DUB MCT 55 45 | Caprylic/Capric Triglyceride | 3 |
| | Lanol 99 | Isononyl isononanoate | 3 |
| | Vegelight Iso-Silk | C9-C12 Alkane | 3.5 |
| C | Microcare PHDG | Phenoxyethanol, Caprylyl glycol, Decvlene glycol | 1 |
| D | Cefalu | Perfum | 0.05 |
| E | Solution 10% citric acid | citric acid | Qsp pH 6 |

Mascara formulation MU-017-022:

This formulation was prepared according to the composition of table 13 following the operating protocol below:

1/ Weigh and mix phase A1,

2/ Add phase A2 and heat at 75-80°C for 25 min,

3/ Add A3 and homogenize for 15 MIN at 2200 rpm at 80°C and add pigments,

4/ Weigh and heat phase B1 at 80°C,

5/ Emulsify B1 in A (1-2min),

6/ Add B2, and let stir for 15 min at 2500 rpm at 80°C,

7/ Cool down very low agitation (1100 rpm) and at RT (no ice),

8/ Add C and adjust pH around 7.3.

[Table 13] mascara formulation MU-017-022

| Phase | Ingredient; supplier | INCI | Weight % |
|---|---|---|---|
| A1 | Demineralized water | Aqua | 72.22 |
| A2 | Beauté by Roquette® DS 112; ROQUETTE | Starch Acetate (and) Hydroxyethyl Cellulose (and) Xanthan Gum | 1 |
| A3 | Invention starch #1 | Not applicable | 5 |
| A4 | Unipure Black LC 998 PHY; SENSIENT | CI 77499 (and) Phytic Acid (and) Sodium Hydroxide | 10 |
| B1 | Beeswax White; KOSTER KEUNEN | Cera Alba | 2 |
| | Carnauba wax T1; KOSTER KEUNEN | Copernicia Cerifera Cera | 1 |
| | Candelilla Wax; KOSTER KEUNEN | Candelilla Cera | 2 |
| | Ricinus communis seed oil; COOPER | Huile Ricin vierge | 2 |
| | Cutina® FS 45; BASF | Stearic Acid (and) Palmitic Acid | 3.5 |
| B2 | SODIUM HYDROXYDE PASTILLES; Cooper | Sodium hydroxyde | 0.28 |
| C | Microcare® PHC; THOR | Phenoxyethanol, Chlorphenesin, Aqua, Glycerin | 1 |

Eyeliner formulation MU-050-002:

This formulation was prepared according to the composition of table 14 following the operating protocol below:

1/ Disperse Avicel PC 611 in water 5-10 minutes and heat at 80°C,

2/ Disperse Bentonite,

3/ Add invention starch #1 under 2200 rpm 15 minutes at 80°C,

4/ Cool down to 22°C,

5/ Add alcohol and preservative and mix 15 minutes.

[Table 14] eyeliner formulation MU-050-002

| Phase | Ingredient; supplier | INCI | Weight % |
|---|---|---|---|
| A | Demineralized water | Aqua | 72.50 |
| | Avicel PC 611; FMC BioPolymer | Microcrystalline cellulose, Cellulose gum | 0.7 |
| | Kunipia-F; Kunimine Industries | Bentonite | 0.8 |
| | Invention starch #1 | Not applicable | 5 |
| B | Unipure Black LC998 PHY; Sensient | CI 77499, Phytic Acid, Sodium Hydroxide | 10 |
| | Absolute Ethyl Alcohol; Cooper | Alcohol | 10 |
| C | Microcare® PHC; Thor | Phenoxyethanol, Chlorphenesin, Aqua, Glycerin | 1 |

Eyeshadow formulation MU-001-014:

This formulation was prepared according to the composition of table 15 following the operating protocol below:

1/ Add ingredients of phase A in water and heat at 80°C during 40 minutes,

2/ Add invention starch #1 and mix during 15min at 80°C,

3/ Prepare phase C : mix and heat at 80°C until homogeneous,

4/ Emulsify Phase B in phase A during 15 minutes,

5/ Cool down to 22°C.

[Table 15] eyeshadow formulation MU-001-014

| Phase | Ingredient; supplier | INCI | Weight % |
|---|---|---|---|
| A | Demineralized water | Aqua | 66.38 |
| | Microcare® PHC; Thor | Phenoxyethanol, Chlorphenesin, Aqua, Glycerin | 1 |
| | Covarine red WN 3798; Sensient | CI 77491, Glycerin, Xanthan gum, Sodium citrate, Aqua | 0.07 |
| | Covarine black WN 9798; Sensient | CI 77499, Glycerin, Xanthan gum, Sodium citrate, Aqua | 0.55 |
| | Glycerin; Cooper | Glycerin | 1 |
| | Beauté by Roquette® DS 112; Roquette | Starch Acetate, Hydroxyethylcellulose, Xanthan Gum | 3 |
| B | Invention starch #1 | | 5 |
| C | Imwitor® 900 K; IOI Oleochemical | Glyceryl Stearate | 1.9 |
| | Imwitor® 372 P; IOI Oleochemical | Glyceryl Stearate Citrate | 1.1 |
| | UNIPURE WHITE LC987; Sensient | CI 77897 | 1.5 |
| | Ricinus communis seed oil; Cooper | Huile de ricin | 5 |
| | LABRAFAC CC MB; Gattefossé | Caprylic/Capric Triglyceride | 5 |
| D | COVAPEARL STAR DORE 2375; Sensient | CI 77891, Synthetic Fluorphlogopite | 2 |
| | COVAPEARL LIGHT BROWN 830; Sensient | CI 77491, Mica | 6.5 |

Eye serum formulation SC-103-009:

This formulation was prepared according to the composition of table 16 following the operating protocol below:

1/ Prepare phase A: Disperse Glycerine in water and start heating to 80°C,

2/ Prepare phase A2: blend Sucraclear V2 and Keltrol® CG-SFT then add to phase A under agitation with deflocculator,

3/ Mix (strong agitation) at 80°C until obtaining a homogeneous gel without lumps,

4/ Add invention starch #1 (A3) and mix for 15 minutes with strong agitation,

5/ Cool down to room temperature (below 25°C),

6/ Add Beauté by Roquette® PO 500 (Phase B) and mix for 5 minutes with medium agitation,

7/ Add preservative,

8/ Add phase D (pearls) until homogeneous dispersion,

9/ Disperse slowly and under strong agitation Beauté by Roquette® ST 118 and keep stirring (low to medium agitation speed) until fully hydrated.

[Table 16] eye serum formulation SC-103-009

| Phase | Ingredient; supplier | INCI | Weight % |
|---|---|---|---|
| A1 | Demineralized water | Aqua | 83.5 |
| | Glycerin; Cooper | Glycerin | 2 |
| A2 | Sucraclear V2; Alchemy ingredients | Cellulose Gum, Chondrus crispus Powder (Carrageenan), Ceratonia siliqua Gum, Glucose | 1 |
| | KELTROL® CG-SFT; CP Kelco | Xanthan Gum | 0.5 |
| A3 | Invention starch #1 | Not applicable | 4 |
| B | Beauté by Roquette® PO 500; Roquette | Isosorbide | 6 |
| C | Microcare® PHC; Thor | Phenoxyethanol, Chlorphenesin, Aqua, Glycerin | 1 |
| D | SynCrystal Gold; Eckart | Synthetic Fluorphlogopite, Titanium Dioxide, Tin Oxide | 0.6 |
| E | Beauté by Roquette® ST 118; Roquette | Sodium Carboxymethyl Starch | 1.4 |

Foundation formulation MU-037-021:

This formulation was prepared according to the composition of table 17 following the operating protocol below:

1/ Prepare phase C (Oil + pigments) under agitation with deflocculator 10-15min in order to disperse pigments,

2/ Mix C + D,

3/ Heat A at 90°C until homogeneous dispersion and after add B,

4/ Add A+B in C+D,

5/ In parallel prepare phase E, disperse invention starch #1 in water at 80°C during 15min at 2000rpm,

6/ Add successively ingredients of phase F in E,

7/ Emulsify very slowly F+E in oily phase (A+B+C+D).

[Table 17] foundation formulation MU-037-021

| Phase | Ingredient; supplier | INCI | Weight % |
|---|---|---|---|
| A | Cetiol® C5; BASF | Coco-Caprylate/Caprate | 9 |
| | Magnesium stearate; Cooper | Magnesium stearate | 2 |
| B | Emulium Illustro; Gattefossé | Polyglyceryl-6 Polyhydroxystearate, Polyglyceryl-6 Polyricinoleate | 5 |
| C | Cetiol OE; BASF | Dicaprylyl Ether | 5 |
| | Unipure White LC981 HLC; Sensient | CI 77891, Hydrogenated Lecithin | 5 |
| | Unipure Yellow LC 182 HLC; Sensient | CI 77492, Hydrogenated Lecithin | 1.2 |
| | Unipure Red LC 381 HLC; Sensient | CI 77491, Hydrogenated Lecithin | 0.2 |
| | Unipure Black LC 989 HLC; Sensient | CI 77499, Hydrogenated Lecithin | 0.1 |
| | Labrafac CC MB; Gattefossé | Caprylic/Capric Triglyceride | 5 |
| D | Harmonie™ SOFT FLUID; Momentive | C-9-12 Alkane | 3 |
| | Beauté by Roquette® ST 012; Roquette | Aluminum Starch Octenylsuccinate | 1 |
| E | Demineralized water | Aqua | 51.5 |
| | Invention starch #1 | Not applicable | 6 |
| | Glycerin; Cooper | Glycerin | 1 |
| F | Usp Magnesium Sulfate, Anhydrous; Kobo | Magnesium Sulfate | 1 |
| | Microcare® PHC; Thor | Phenoxyethanol, Chlorphenesin, Aqua, Glycerin | 1 |
| | Sodium Chlorure; Cooper | Sodium Chloride | 1 |

Tightening serum formulation SC-105-004:

This formulation was prepared according to the composition of table 18 following the operating protocol below:

1/ Disperse successively ingredients of phase A2 in phase A1 and heat at 80°C at the same time,

2/ Disperse invention starch #1 at 80°C during 15 min,

3/ Heat phase C at 80°C and emulsify in water phase A1+A2+B during 10min at 80°C,

4/ Cool down and add D then E.

[Table 18] tightening serum formulation SC-105-004

| Phase | Ingredient; supplier | INCI | Weight % |
|---|---|---|---|
| A1 | Demineralized water | Aqua | 81.1 |

(continued)

| Phase | Ingredient; supplier | INCI | Weight % |
|---|---|---|---|
| A2 | Glycerin; Cooper | Glycerin | 2 |
| | Cosmedia® SP; BASF | Sodium Polyacrylate | 0.2 |
| | KELTROL® CG-SFT; CP Kelco | Xanthan Gum | 0.1 |
| B | Invention starch #1 | Not applicable | 4 |
| C | Eumulgin® SG; BASF | Sodium Stearoyl Glutamate | 0.1 |
| | Tsubaki oil; Jan Dekker | Camellia Japonica Seed Oil | 5 |
| | Imwitor® 960K; IOI Oleochemical | Glyceryl stearate | 0.5 |
| D | Beauté by Roquette® PO 500; Roquette | Isosorbide | 6 |
| E | Microcare® PHC; Thor | Phenoxyethanol, Chlorphenesin, Aqua, Glycerin | 1 |

Tightening white cream formulation SC-106-008:

This formulation was prepared according to the composition of table 19 following the operating protocol below:

1/Prepare phase A, disperse thickener in water without agitation and heat at 80°C at the same time,

2/ Under agitation disperse invention starch #1 at 80°C during 15 min (2200 rpm),

3/ Heat phase C at 80°C and emulsify in water phase during 10min at 80°C,

4/ Cool down and add Beauté by Roquette® ST 118 until homogeneous aspect,

5/ Add E then F.

[Table 19] tightening white cream formulation SC-106-008

| Phase | Ingredient; supplier | INCI | Weight % |
|---|---|---|---|
| A | Demineralized water | Aqua | 87.64 |
| | CARBOPOL® ULTREZ 10 POLYMER; Lubrizol | Carbomer | 0.2 |
| B | Invention starch #1 | Not applicable | 4 |
| C | Eumulgin® SG; BASF | Sodium Stearoyl Glutamate | 0.1 |
| | Tsubaki oil; Jan Dekker | Camellia Japonica Seed Oil | 5 |
| | Imwitor® 960K; IOI Oleochemical | Glyceryl stearate | 0.5 |
| D | Beauté by Roquette® ST 118; Roquette | Sodium Carboxymethyl Starch | 1.5 |
| E | Microcare® PHC; Thor | Phenoxyethanol, Chlorphenesin, Aqua, Glycerin | 1 |
| F | SODIUM HYDROXYDE PASTILLES; Cooper | Sodium hydroxyde | 0.06 |

Tightening green cream formulation SC-106-002:

This formulation was prepared according to the composition of table 20 following the operating protocol below:

1/ Prepare phase A1, disperse glycerin,

2/ And thickener A2 in water phase A1 without agitation for hydration and heat at 80°C at the same time,

3/ Disperse A3 under agitation in phase A1+A2,

4/ Under agitation disperse invention starch #1 at 80°C during 15 min (2200 rpm) in phase A1 +A2+A3,

5/ Heat phase C at 80°C and emulsify in water phase A1+A2+A3+B during 10m in at 80°C,

6/ Cool down and add successively D, E and F.

[Table 20] tightening green cream formulation SC-106-002

| Phase | Ingredient; supplier | INCI | Weight % |
|---|---|---|---|
| A1 | Demineralized water | Aqua | 86.937 |
| | Glycerin | Glycerin | 2 |
| A2 | CARBOPOL® ULTREZ 10 POLYMER | Carbomer | 0.3 |
| A3 | KELTROL® CG-SFT | Xanthan Gum | 0.1 |
| B | Invention starch #1 | Not applicable | 4 |
| C | Eumulgin® SG | Sodium Stearoyl Glutamate | 0.1 |
| | Tsubaki oil | Camellia Japonica Seed Oil | 5 |
| | Imwitor® 960K | Glyceryl stearate | 0.5 |
| D | Unicert Yellow 08005-J | CI 19140 | 0.0018 |
| | UNICERT BLUE 05601-J | CI 42090 | 0.0012 |
| E | Microcare® PHC | Phenoxyethanol, Chlorphenesin, Aqua, Glycerin | 1 |
| F | SODIUM HYDROXYDE PASTILLES | Sodium hydroxyde | 0.06 |

Sun care cream formulation SU-003-006:

This formulation was prepared according to the composition of table 21 following the operating protocol below:

1/ Disperse xanthan gum in aqua and heat until 80°C add A2 during 15min,

2/ Prepare phase B1 and mix with magnetic agitation at 80°C,

3/ Prepare phase B2 and mix with magnetic agitation at room temperature,

4/ Mix phases B1 and B2 and Emulsify phase B in phase A with a strong stirring (3000rpm during 10mins),

5/ Cool down at room temperature and add phase C,

6/ Add phase D at pH 5,5.

[Table 21] sun care cream formulation SU-003-006

| Phase | Ingredient; supplier | INCI | Weight % |
|---|---|---|---|
| A1 | Demineralized water | Aqua | 55.68 |
| | KELTROL CG-SFT; CP Kelco | Xanthan Gum | 0.3 |
| A2 | Invention starch #1 | Not applicable | 5 |

(continued)

| Phase | Ingredient; supplier | INCI | Weight % |
|---|---|---|---|
| B1 | Montanov L; SEPPIC | C14-22 Alcohols (and) C12-20 Alkyl glucoside | 4 |
| | Tsubaki oil; Jan Dekker | Camellia Japonica Seed Oil | 8 |
| | Tocopherol; BTSA | BIOXAN®SF T 50 | 0.02 |
| B2 | Eusolex 2292; Rona/Merck | Ethylhexyl Methoxycinnamate | 6 |
| | Eusolex HMS; Rona/Merck | Homosalate | 5 |
| | Eusolex OCR; Rona/Merck | Octocrylene | 3 |
| | Tinosorb S; BASF | Bis-Ethylhexyl Methoxyphenyl Triazine | 4 |
| C | Microcare PHC; Thor | Phenoxyethanom (and) Chlorphenesin (and) Glycerin | 1 |
| D | Tinosorb M in solution with acetic acid (pH 5,5); BASF | Methylene Bis-Benzotriazolyl Tetramethylbutylphenol | 8 |

Sun care cream formulation SU-024-014 V2:

This formulation was prepared according to the composition of table 22 following the operating protocol below:

1/ Prepare phase A and disperse Bentonite in the beaker until homogeneous solution,

2/ Then disperse Xanthan gum, heat at 80°C and add invention starch #1 (15min),

3/ Prepare phase B and heat at 80°C,

4/ Emulsify oily phase B in aqueous phase under strong agitation (3000rpm for 10 mins),

5/ Cool down at room temperature (20-25°C),

6/ Add Phase C,

7/ Adjust the pH at 7,5 if necessary.

[Table 22] sun care cream formulation SU-024-014 V2

| Phase | Ingredient; supplier | INCI | Weight % |
|---|---|---|---|
| A1 | Demineralized water | Aqua | 27 |
| | Beauté by Roquette® GA 080; Roquette | Sodium Gluconate | 0.2 |
| | Kunipia-F; Kunimine Industries | Bentonite | 1 |
| A2 | KELTROL® CG-SFT; CP Kelco | Xanthan Gum | 0.3 |
| A3 | Invention starch #1 | Not applicable | 5 |

(continued)

| Phase | Ingredient; supplier | INCI | Weight % |
|---|---|---|---|
| B | Imwitor 900K ou 960K; IOI Oleochemical | Glyceryl stearate | 2 |
| | imwitor 372P; IOI Oleochemical | Glyceryl stearate citrate | 1 |
| | Zano® 20 plus; EverCare | Zinc Oxide, Triethoxycaprylyl silane | 25 |
| | UV Cut TiO2-55-CG; Grant Industries | Titanium Dioxide, Caprylic/Capric Triglyceride, Polyhydroxystearic Acid, Stearic Acid, Alumina | 15 |
| | LexFeel™ N200 MB; Inolex | Diheptyl Succinate, Capryloyl Glycerin/Sebacic Acid Copolymer | 5 |
| | Refined sweet almond oil; Amazon | Prunus Amygdalus Dulcis Oil | 4 |
| | BIOXAN®SF T 50; BTSA | Tocopherol | 0.5 |
| | Dub VCI 10; Stéarinerie Dubois | Isodecyl Neopentanoate | 5 |
| | WITARIX® MCT 60/40; IOI Oleochemical | Caprylic/Capric Triglyceride | 7.5 |
| | Dispersun DSP-OL100; Innospec | Polyhydroxystearic Acid | 0.5 |
| C | Microcare® PHC; Thor | Phenoxyethanol, Chlorphenesin, Aqua, Glycerin | 1 |

## Citation List

### Non-Patent Literature

For any purpose, the following non-patent element(s) is (are) cited:

- nplcit1: Roy L. Whistler and Eugene F. Paschall, « Starch: Chemistry and Technology, Volume II Industrial aspects »; Academic Press, 1967, 733 p. (library of congress catalog card number:65-21330);

- nplcit2: https://www.andritz.com/products-en/separation/contact-dryers/gouda-drum-dryer;

## Claims

1. **Pregelatinized hydrophilically modified legume starch** having a RVA viscosity profile **characterized by** a final cold viscosity from 100 to 2000 cPo, or 125 to 1750 cPo, or 150 to 1500 cPo, or to 200 to 1200 cPo, the RVA temperature and stirring profile being the following:

   - at time= 0 s: temperature= 25°C; stirring speed= 100 rpm,
   - at time= 10 s: temperature= 25°C; stirring speed= 500 rpm,
   - at time= 30 s: temperature= 25°C; stirring speed= 160 rpm,
   - at time= 10 min: temperature= 25°C; stirring speed= 160 rpm,
   - at time= 18 min: temperature= 90°C; stirring speed= 160 rpm,
   - at time= 21 min: temperature= 90°C; stirring speed= 160 rpm,
   - at time=29 min: temperature= 30°C; stirring speed= 160 rpm,
   - at time=34 min: temperature= 30°C; stirring speed= 160 rpm.

2. Starch according to claim 1, wherein its RVA viscosity profile is **characterized by** an initial cold viscosity from 250 to 4000 cPo, or from 400 to 3500 cPo, or from 500 to 3000 cPo.

3. Starch according to any of claims 1 or 2, wherein the legume starch has an amylose content greater than or equal to 20 wt%, or 24 wt%, or 30 wt%, or 34 wt%, preferentially from 30 to 40 wt%.

4. Starch according to claim 3, where the legume starch is chosen from pea starch, bean starch, fava bean starch, faba bean starch, lentil starch, alfalfa starch, clover starch, lupin starch, or soybean starch, preferentially is a pea starch.

5. Starch according to any of claims 1 to 4, wherein the starch is hydroxyalkylated and/or carboxyalkylated, preferentially is hydroxyalkylated.

6. Starch according to any of claims 1 to 5, wherein the degree of substitution is from 0,01 to 3, or from 0,05 to 0,8, or from 0,05 to 0,6, or from 0,05 to 0,21, preferentially 0,15 to 0,19.

7. Starch according to any of claims 1 to 6, wherein the starch has a solubility in water at 22°C from 25 to 80 %, or from 30 to 75%, or from 35 to 60%, or from 45 to 55%.

8. Starch according to any of claims 1 to 7, wherein the particle size distribution of the starch has a volume-average mean diameter D(4,3) from 25 to 200 microns, or from 40 to 150 microns, or from 50 to 100 microns.

9. Starch according to any claims 1 to 8, having a hydrophobicity value as measured according to the model of Young-Laplace and Fowkes equal to or greater than 30%, or 35%, preferentially from 30 to 55%, or from 35 to 50%.

10. of manufacturing of a pregelatinized hydrophilically modified legume starch as described in any of claims 1 to 13, comprising, preferentially consisting of, the steps of:

    a) providing a native legume starch,
    b) hydrophilically modifying, preferentially hydroxypropylating, the native legume starch of step a,
    c) pregelatinizing the hydrophilically modified legume starch of step b,
    d) grinding the pregelatinized hydrophilically modified legume starch of step c).

11. Process according to claim 11, wherein the pregelatinizing step is performed using a drum dryer or an extruder, or a combination of a jet cooker and a spray-dryer, preferentially is performed using a drum dryer.

12. of a pregelatinized hydrophilically modified legume starch as described in any of claims 1 to 13, in a formulated product, wherein the starch is a water-resistant film former.

13. Use according to claim 12, wherein the cosmetic product is chosen from a skin care product, a hair care product, a make-up product, an oral care product, or a hygiene product.

14. comprising a pregelatinized hydrophilically modified legume starch as described in any of claims 1 to 9, in a physiologically acceptable medium.

15. Film forming composition according to claim 14, wherein the content in the pregelatinized hydrophilically modified legume starch is from 0,5 to 10 weight % of the total weight of the film forming composition, or from 3 to 7 weight %, or from 4 to 6 weight %.

16. **Film formed on the surface** of a human or animal skin, mucous membrane, or hair, comprising, or consisting of, a pregelatinized hydrophilically modified legume starch as described in any of the claims 1 to 9, or obtained by the application of a film forming composition as described in any of claims 14 or 15.

17. Film according to claim 16, having a resistance to water equal to or higher than 1 minute according to test A, or equal to or higher than 2 minutes, or equal to or higher than 3 minutes, or equal to or higher than 4 minutes, or equal to or higher than 5 minutes.

18. Film according to any of claims 16 or 17, having a contact angle with a drop of water as measured by goniometry, equal to or higher than 70° after 1 minute of contact between the drop and the film, and/or 60° after 5 minutes of contact between the drop and the film.

[Fig. 1] Photos of water resistance on glass plates

[Fig. 2] Photos of water resistance on bandage

[Fig. 3] Photos of water resistance on synthetic lashes

Europäisches
Patentamt

European
Patent Office

Office européen
des brevets

**EUROPEAN SEARCH REPORT**

**Application Number**

EP 24 30 5421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/110799 A1 (LEFERVE PHILIPPE [FR] ET AL) 17 May 2007 (2007-05-17) * [0097] * | 1-18 | INV. C08B30/14 C08B31/12 C08L3/08 A61K8/73 |
| X | US 2013/289055 A1 (BOIT BAPTISTE [FR] ET AL) 31 October 2013 (2013-10-31) * [0103] * | 1-18 | |

**TECHNICAL FIELDS SEARCHED (IPC)**

C08B
C08L
A61Q
A61K

The present search report has been drawn up for all claims

| Place of search | Date of completion of the search | Examiner |
|---|---|---|
| Munich | 26 July 2024 | Pellegrini, Paolo |

EPO FORM 1503 03.82 (P04C01)

EP 4 620 982 A1

**ANNEX TO THE EUROPEAN SEARCH REPORT
ON EUROPEAN PATENT APPLICATION NO.**

EP 24 30 5421

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2024

| Patent document cited in search report | Publication date | Patent family member(s) | Publication date |
|---|---|---|---|
| US 2007110799 A1 | 17-05-2007 | AU 2004305266 A1 | 07-07-2005 |
| | | BR PI0416837 A | 13-02-2007 |
| | | CA 2545961 A1 | 07-07-2005 |
| | | CN 1882317 A | 20-12-2006 |
| | | DK 1684731 T3 | 15-10-2012 |
| | | EP 1684731 A1 | 02-08-2006 |
| | | ES 2390584 T3 | 14-11-2012 |
| | | FR 2862654 A1 | 27-05-2005 |
| | | JP 4890260 B2 | 07-03-2012 |
| | | JP 2007511584 A | 10-05-2007 |
| | | KR 20060132586 A | 21-12-2006 |
| | | MX PA06005703 A | 17-08-2006 |
| | | US 2007110799 A1 | 17-05-2007 |
| | | WO 2005060944 A1 | 07-07-2005 |
| US 2013289055 A1 | 31-10-2013 | BR 112013010491 A2 | 10-10-2017 |
| | | CA 2814184 A1 | 10-05-2012 |
| | | CN 103189434 A | 03-07-2013 |
| | | EP 2635632 A1 | 11-09-2013 |
| | | ES 2726453 T3 | 04-10-2019 |
| | | FR 2966828 A1 | 04-05-2012 |
| | | JP 5919290 B2 | 18-05-2016 |
| | | JP 2014504305 A | 20-02-2014 |
| | | KR 20140001207 A | 06-01-2014 |
| | | MX 339755 B | 08-06-2016 |
| | | RU 2013125503 A | 10-12-2014 |
| | | US 2013289055 A1 | 31-10-2013 |
| | | WO 2012059689 A1 | 10-05-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020128253 A
- WO 2020201071 A
- WO 2022199889 A

**Non-patent literature cited in the description**

- **R. HOOVER et al.** Composition, structure, functionality and chemical modification of legume starches: a review. *Can. J. Physiol. Pharmacol.*, 1991, vol. 69, 79-92
- **C-L HEYDLEY et al.** Developing novel pea starches. *Proceedings of the Symposium of the Industrial Biochemistry and Biotechnology Group of the Biochemical Society*, 1996, 77-87
- **WHISTLER** ; **PASCHALL**. Starch: Chemistry and Technology, volume II Industrial aspects. 1967, 523-536
- **ROY L. WHISTLER** ; **EUGENE F. PASCHALL**. Starch: Chemistry and Technology, Volume II Industrial aspects. Academic Press, 1967, 733